# EUROPEAN PATENT SPECIFICATION

(11) **EP 3 065 833 B1**
(45) Date of publication and mention of the grant of the patent: **30.01.2019**
(21) Application number: 14781185.5
(22) Date of filing: 07.10.2014
(51) Int. Cl.: A61Q 11/00, A61K 8/02, A61K 8/19

(54) **COMPOSITIONS AND METHODS FOR TREATING TOOTH HYPERSENSITIVITY**
ZUSAMMENSETZUNGEN UND VERFAHREN ZUR BEHANDLUNG VON ZAHNHYPERSENSIBILITÄT
COMPOSITIONS ET PROCÉDÉS PERMETTANT DE TRAITER L'HYPERSENSIBILITÉ DENTAIRE

(30) Priority: 07.11.2013 EP 13191861
(43) Date of publication of application: 14.09.2016
(73) Proprietor: Unilever PLC, London, Greater London EC4Y 0DY (GB); Unilever N.V., 3013 AL Rotterdam (NL)
(72) Inventor: ASHCROFT, Alexander Thomas, Wirral Merseyside CH63 3JW (GB); DWYER-JOYCE, Robert Sean, Sheffield South Yorkshire S1 3JD (GB); FAIRLEY, Peter, Wirral Merseyside CH63 3JW (GB); GREEN, Alison Katharine, Wirral Merseyside CH63 3JW (GB); GROVES, Brian Joseph, Wirral Merseyside CH63 3JW (GB); ROSE, Christopher Edward, Sheffield South Yorkshire S1 3JD (GB)
(74) Representative: Tansley, Sally Elizabeth
(86) International application number: PCT/EP2014/071383
(87) International publication number: WO 2015/067422

(56) References cited:
- WO-A1-2013/041419
- CN-A- 101 889 964
- GB-A- 2 481 630
- US-A1- 2012 027 828
- US-A1- 2013 224 270

## Description

### Field of the Invention

The present invention relates to compositions and methods for treating tooth hypersensitivity using certain particulate calcium carbonates.

### Background and Prior Art

Tooth hypersensitivity is a common but painful condition affecting up to 20% of the adult population. Hypersensitive teeth may be sensitive to cold, heat, air or sugary foods.

The incidence of tooth hypersensitivity increases with age. Tooth hypersensitivity is believed to be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed. Dentinal tubules are naturally present in the dentinal layer of the tooth and they provide for an osmotic flow between the inner pulp region of the tooth and the outer root surfaces.

The currently accepted theory for tooth hypersensitivity is the hydrodynamic theory, based on the belief that open exposed dentinal tubules allow fluid flow through the tubules. This flow excites the nerve endings in the dental pulp. Clinical replicas of sensitive teeth viewed in a scanning electron microscope (SEM) reveal varying numbers of open or partially occluded dentinal tubules.

There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

CN101889964A discloses desensitizing toothpaste comprising adhesive, foaming agent, humectant, sweetening agent, friction agent, flavors, de-ionized water, honeysuckle extract, mugwort leaf.

US2013/224270 discloses desensitizing toothpastes comprising comprising a basic amino acid or salt thereof, and an abrasive system comprising natural calcium carbonate and precipitated calcium carbonate.

An example of an occluding agent is found in US 5,270,031 which describes a tubule occluding desensitizer comprising a polyacrylic acid such as Carbopol® polymeric materials. Another tubule occluding composition is disclosed in US 5,374,417 which discloses a potassium salt of a synthetic anionic polymer, such as a polycarboxylate.

There is a continuing need for desensitizing agents which can provide treatment and relief of hypersensitivity whilst maintaining a pleasing "mouth-feel" for the product in which they are formulated.

There is also a need for products which are effective for the treatment of hypersensitivity, but which do not require excessively frequent product application or long treatment times and which can be accomplished by a consumer without intervention of a dentist.

The present inventors have found that certain acicular calcium carbonates of specific particle size are effective as dentinal tubule occluding agents. Furthermore, they can provide a smooth and pleasant mouth-feel when formulated into products such as dentifrices. This is particularly advantageous in the context of dental hypersensitivity treatment since it encourages regular and repeated product use by the consumer, which reinforces the anti-hypersensitivity benefit achieved.

Additionally, by reacting with phosphates (such as those naturally found in saliva), the calcium carbonate particles may assist with strengthening or remineralising enamel or dentine, and may help to form plugs in tubules which further reduce sensitivity.

Acicular calcium carbonates have been proposed in the literature as a dentifrice component. However, there is no suggestion that these materials would be effective in the context now described, namely the treatment of hypersensitivity arising in the natural human tooth, and in particular the occlusion of dentinal tubules.

### Summary of the Invention

The present invention provides an anhydrous oral care dentifrice composition comprising 60 to 75% glycerol (by weight based on the total weight of the dentifrice) and a particulate calcium carbonate composed of primary particles which are acicular and which have a length of 2 microns or greater, the composition being adapted to be applied to the surface of the teeth in a mixture with water; for the treatment of hypersensitivity arising in natural human teeth.

The invention also relates to a method of treating hypersensitivity arising in natural human teeth, the method comprising mixing an anhydrous oral care composition as described above with water outside of the mouth, and applying the mixture so obtained to the surface of the teeth.

The invention also relates to the use of particulate calcium carbonate composed of primary particles which are acicular and which have a length of 2 microns or greater; for the treatment of hypersensitivity arising in natural human teeth.

### Detailed Description of the Invention

The anhydrous oral care composition of the invention comprises a particulate calcium carbonate composed of primary particles which are acicular and which have a length of 2 microns or greater.

By "primary particles" is meant individual particles. For the purposes of the present invention, the primary particles of the particulate calcium carbonate abrasive are acicular and have a length of 2 microns or greater.

The primary particles may associate under certain conditions to form larger secondary structures such as aggregates or agglomerates.

If desired, the primary particles may be surface-modified, for example by coating with hydrophobic materials such as ethylcellulose, hydroxypropylcellulose, waxes (such as shellac, carnauba or beeswax) and fat or fatty acids such as stearic and oleic acid. Coating can be done using colloidal precipitation using solvent or temperature change, for instance.

For the purposes of the present invention, a suitable class of particulate calcium carbonate abrasive includes crystalline calcium carbonates in which the crystals are acicular and have a length of 2 microns or greater.

The term "crystal" means an essentially fully dense solid composed of atoms arranged in an orderly repetitive array bounded by plane surfaces which are the external expression of internal structure.

Calcium carbonate may occur naturally or may be synthetically produced in three particular crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.

The term "acicular" in this context refers to the shape of the crystals. Usually, crystals grow in three directions, length, width and height. Some crystals however, have one or two preferred growth directions. Acicular crystals have a preferred crystal growth in one direction. Examples are crystals in the form of needles, rods, fibres, whiskers or columns and the like. For the purposes of the present invention, preferred acicular crystals are rod-like or needle-like with a generally uniform, typically generally circular, cross-sectional shape.

The ratio between the length and the width of a crystal, the so-called aspect ratio, is higher than 1:1 for acicular crystals. The higher the aspect ratio, the longer the crystal. For the purposes of the present invention the aspect ratio is preferably at least 2.5:1, more preferably at least 10:1. For example the aspect ratio may typically range from 2.5:1 to 200:1, and preferably ranges from 10:1 to 60:1, more preferably from 20:1 to 30:1.

Acicular crystalline forms of calcium carbonate are available naturally, or may be produced by precipitation production technology. Typically, precipitated calcium carbonate is prepared by exposing calcium hydroxide slurry to a carbonation reaction.

Preferred acicular calcium carbonate crystals for use in the invention have a length ranging from 2 to 100 microns, more preferably from 10 to 30 microns and a width ranging from 0.1 to 4.0 microns, more preferably from 0.5 to 1.0 microns.

Crystal morphology and structure may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particle and agglomerate sizes.

Automated image analysis using computer software enables the user to determine particle size distributions. Scanning electron microscopy (SEM) is a particle counting technique and produces a number-weighted size distribution. Accordingly the figures quoted herein for particle length and width will generally represent average values over a population of particles, more specifically the D[1,0] number-length mean of the particle length or the particle width respectively.

A specific class of material suitable for use in the invention includes aragonite crystal form calcium carbonates such as those described for example in US 5,164,172. According to US 5,164,172, aragonite crystal form calcium carbonate with an acicular shape is prepared by premixing aragonite calcium carbonate and Ca(OH)₂ to prepare an aqueous slurry, adding a water-soluble phosphoric acid compound (such as phosphoric acid or a water-soluble salt thereof) into the aqueous slurry, and introducing CO₂ gas into the aqueous slurry to cause a carbonation reaction to take place. In this process, the molar ratio of the aragonite calcium carbonate to Ca(OH)₂ slurry is preferably 1:7 to 1:5000. The resulting aragonite calcium carbonate crystals have an acicular shape and have a particle size of 10 to 100 microns (length) by 0.5 to 4.0 microns (width). The exemplified crystals are shown by microscopy to be rod-like or needle-like with a length of 10 to 100 microns. The rods or needles are generally uniform and typically circular in cross-section, with a cross-sectional diameter of 0.5 to 4.0 microns. For the purposes of the present invention, the most preferred aragonite calcium carbonate crystals are rod-like or needle-like as described above, with a length ranging from 10 to 30 microns, a width ranging from 0.5 to 1.0 microns and an aspect ratio ranging from 20:1 to 30:1.

Suitable aragonite crystal form calcium carbonates for use in the invention are commercially available and include those marketed by Maruo Calcium Company Limited, Japan under the trade name WHISCAL®.

Mixtures of any of the above described materials may also be used.

In order to treat hypersensitivity arising in natural human teeth, the particulate calcium carbonate as defined above (hereinafter termed "acicular calcium carbonate") is incorporated into an anhydrous oral care composition which is adapted to be applied to the surface of the teeth in a mixture with water.

The term "anhydrous" in the context of the present invention generally means that water is not deliberately added to the composition in any significant quantity. However, the term "anhydrous" does not mean that small amounts of water cannot be present, for example as a consequence of its association with hygroscopic raw materials. Accordingly, for the purposes of this invention, the term "anhydrous" generally means that water is present in an amount no greater than about 5 wt%, more preferably no greater than about 3 wt%, most preferably no greater than about 1 wt% (by weight based on the total weight of the composition).

The relative amount of acicular calcium carbonate to be incorporated into the anhydrous oral care composition will depend to some extent on the type of composition used and its typical mode of use (for example typical product dosage and the amount of time it stays in contact with the affected site).

The anhydrous oral care composition may take any product form suitable for application to the surface of the teeth in a mixture with water. Examples of such product forms include solid forms, such as powders or discrete unit doses (for example pellets, pastilles, tablets and the like).

The anhydrous oral care composition is in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

A dentifrice for use in the invention is suitably in the form of an extrudable semi-solid such as a cream, paste or gel (or mixture thereof).

The viscosity of the dentifrice generally ranges from 10,000 to 100,000 cps, and preferably ranges from 30,000 to 60,000 cps, (when measured at 25 °C on a Brookfield viscometer).

The pH of the dentifrice generally ranges from 7 to 10.5, and preferably ranges from 8 to 9, when measured at 25° C using conventional pH sensitive electrodes.

The level of acicular calcium carbonate incorporated into the dentifrice generally ranges from 10 to 70 wt%, preferably from 20 to 60 wt%, more preferably from 30 to 40 wt%, by weight based on the total weight of the dentifrice.

A dentifrice for use in the invention will typically contain a anhydrous liquid continuous phase in an amount of from 40 to 99 wt%, by weight based on the total weight of the dentifrice.

Such an anhydrous liquid continuous phase will preferably comprise one or more organic polyols having 3 or more hydroxyl groups in the molecule (hereinafter termed "organic polyols"), with the amount of organic polyol generally ranging from 20 to 90 wt%, preferably from 35 to 90 wt%, more preferably from 45 to 80 wt% (by total weight organic polyol based on the total weight of the dentifrice). Preferred organic polyols having 3 or more hydroxyl groups in the molecule for use in the invention include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The dentifrice for use in the invention is most preferably organic polyol-based. In the context of the present invention, the term "organic polyol-based" generally means that the dentifrice is not oil-based or water-based, but instead organic polyol (as defined above) forms a liquid continuous phase in which the particulate ingredients of the dentifrice (such as the acicular calcium carbonate) are dispersed. The dentifrice for use in the invention is glycerol-based (i.e. glycerol forms a liquid continuous phase in which the particulate ingredients of the dentifrice are dispersed), and contains from 60 to 75 wt% glycerol (by weight based on the total weight of the dentifrice).

A dentifrice for use in the invention will also generally contain further ingredients to enhance performance and/or consumer acceptability.

For example, the dentifrice may suitably comprise one or more surfactants in an amount of from 0.2 to 5wt% (by weight based on the total weight of the oral care composition) Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Preferred surfactants for use in the invention are selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof. Mixtures of any of the above described materials may also be used.

The dentifrice may also include one or more thickening agents to provide a desirable rheology and texture. Suitable thickening agents for use in this context may be selected from natural or synthetic organic thickeners or gelling agents. Examples of such materials include carboxyvinyl polymers (such as polyacrylic acids cross-linked with polyallyl sucrose or polyallyl pentaerythritol), hydroxyalkyl cellulose derivatives (such as hydroxyethyl cellulose, hydroxypropyl cellulose, hydroxyethylpropyl cellulose, hydroxybutylmethyl cellulose and hydroxypropylmethyl cellulose), water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), starch and polyvinylpyrrolidone. Inorganic thickeners such as finely divided silicas, hectorites and colloidal magnesium aluminium silicates may also be used.

Mixtures of any of the above described materials may also be used.

The amount of thickening agent incorporated into the dentifrice will depend on the particular agent (or agents) used, but generally ranges from about 0.05 to about 10 wt%, preferably from about 0.1 to about 2 wt%, by total weight thickening agent based on the total weight of the solid matrix.

An example of a preferred thickening agent for incorporation into the dentifrice is xanthan gum.

Xanthan gum is a fermentation product prepared by action of the bacteria of the genus *Xanthomonas* upon carbohydrates. Four species of *Xanthomonas,* namely *X.campestris, X. phaseoli, X.malvocearum* and *X.carotae* are reported in the literature to be the most efficient gum producers.

Xanthan gum may be generally characterised as an anionic heteropolysaccharide, with a primary structure consisting of repeating pentasaccharide units formed by two glucose units, two mannose units, and a glucuronic acid unit. These repeating pentasaccharide units give xanthan gum its characteristic backbone, which consists of (1 4) • -D-glucopyranosyl units substituted at C-3 on every other glucose residue with a charged trisaccharide sidechain. The trisaccharide sidechain consists of a D-glucuronic acid unit between 2 D-mannose units. Slightly less than half (about 40%) of the terminal D-mannose residues contain a pyruvic acid residue linked via keto groups to the four and six positions, and the D-mannose linked to the main chain mostly contains an acetyl group at position O-6. Some side chains may be missing. The acetate and pyruvate contents are variable on the side chain, and depend on the bacterial strains and on the fermentation conditions used to produce the gum.

Xanthan gum generally has a molecular weight of from 1 million to 50 million. Its viscosity generally ranges from 850 to 1,700 mPa.s (when measured at 25°C using a 1% solution of the gum in 1% KCl, on a viscometer of the Brookfield LV type, at 60 rpm using Spindle No. 3).

Xanthan gum is available from several commercial suppliers such a
RT Vanderbilt Company and CP Kelco. Examples of suitable xanthan gums are Keltrol®, Keltrol® F, Keltrol® T, Keltrol® TF, Xantural® 180 and Vanzan® NF.

The amount of xanthan gum in the dentifrice preferably ranges from 0.1 to 0.9 wt% (by weight based on the total weight of the dentifrice).

An anhydrous oral care composition for use in the invention (such as the dentifrice as described above) may also contain further optional ingredients customary in the art such as anticalculus agents, buffers, flavouring agents, sweetening agents, colouring agents, opacifying agents, preservatives, antisensitivity agents, antimicrobial agents and the like.

### Mode of Use

According to the method of the invention, hypersensitivity arising in natural human teeth is treated by mixing an anhydrous oral care composition (as described above) with water, and applying the mixture so obtained to the surface of the teeth.

The optimum weight ratio of water to anhydrous oral care composition in the above mixture will depend to some extent on the type of anhydrous oral care composition used, but may generally range from about 5:1 to 1:100 (water:anhydrous oral care composition).

Good results have been observed when the anhydrous oral care composition is a glycerol-based dentifrice containing from 60 to 75 wt% glycerol (by weight based on the total weight of the dentifrice) and from 30 to 40 wt% acicular calcium carbonate (by weight based on the total weight of the dentifrice); and the weight ratio in the mixture ranges from 5:1 to 1:100, preferably from 3:1 to 1:3, more preferably from 2:1 to 1:2 and especially about 1:1 (water:anhydrous glycerol based dentifrice).

For carrying out the method of the invention, an anhydrous oral care composition as described above may be supplied to the consumer together with instructions to mix the composition with water and to apply the mixture so obtained to the surface of the teeth.

Alternatively, a kit may be supplied to the consumer in which the anhydrous oral care composition as described above is packaged together with an independent aqueous activator.

The aqueous activator may be any formulation capable of mixing with the anhydrous oral care composition and providing a source of water. The aqueous activator may suitably have an aqueous continuous phase, and will generally comprise at least 50 wt%, preferably at least 70 wt%, more preferably at least 90 wt% water (by weight based on the total weight of the aqueous activator).

An aqueous activator suitable for use in the invention may also contain further optional ingredients customary in the art such as thickeners, anticalculus agents, buffers, flavouring agents, sweetening agents, colouring agents, opacifying agents, preservatives, antisensitivity agents, antimicrobial agents and the like.

In the packaging of a kit as described above, any convenient means for effecting the separation of the anhydrous oral care composition from the aqueous activator before use may be used. For example, a single container may be compartmentalized so that the anhydrous oral care composition and the aqueous activator are housed in separate compartments. Alternatively, the anhydrous oral care composition and the aqueous activator may be housed in physically separate containers (e.g. tubes).

According to the method of the invention, the anhydrous oral care composition is mixed with water, and the mixture so obtained is applied to the surface of the teeth.

The mixing of the anhydrous oral care composition with the water may be carried out in a suitable mixing vessel, and the resulting mixture applied to the surface of the teeth. The mixture will generally be applied to the surface of the teeth via an implement such as a toothbrush, or alternatively by means of an applicator device which is adapted to be placed in the mouth. A suitable form of applicator device in this context comprises a front wall, the inner face of which is adapted in use to contact the front face of the teeth, a bridge portion which is adapted in use to contact the biting edge of the teeth and a rear wall, the inner face of which is adapted in use to contact the rear face of the teeth. Typically the device is generally U-shaped defining a U-shaped channel for receiving the dental arch. The mixture may for example be dispensed into the applicator device, (typically into the U-shaped channel for receiving the dental arch as described above). Alternatively the anhydrous oral care composition and the water (or source thereof) may be separately dispensed into the applicator device and then mixed. The applicator device may then be placed into the mouth to enable contact between the mixture with the surface of the teeth.

The anhydrous oral care composition and the water (or source thereof) may also be separately applied to an implement such as a toothbrush, and mixing effected via the agitation of the implement against the surface of the teeth. This may in some cases be preferred, since it enables simultaneous mixing and application of the anhydrous oral care composition and the water (or source thereof).

Alternatively, naturally occurring saliva may serve as a source of water, which is mixed with the anhydrous oral care composition when the anhydrous oral care composition composition is delivered or applied to the surface of the teeth and agitated against the surface of the teeth. (typically via an implement such as a toothbrush). In such a case, it is preferred that the anhydrous oral care composition is a glycerol-based dentifrice (as described above) containing from 60 to 75 wt% glycerol (by weight based on the total weight of the dentifrice) and from 30 to 40 wt% acicular calcium carbonate (by weight based on the total weight of the dentifrice).

The invention is further illustrated with reference to the following, non-limiting Examples:

### EXAMPLES

The following paste formulations (Examples 1 and 2) illustrate anhydrous oral care compositions according to the invention:

| **Ingredient** | **(wt%)** | |
|---|---|---|
| | **Ex.1** | **Ex.2** |
| Glycerol | 66.5 | 65.5 |
| Xanthan gum | 0.3 | 0.3 |
| Sodium saccharin | 0.2 | 0.2 |
| Flavour | 1.2 | 1.2 |
| Acicular calcium carbonate⁽¹⁾ | 30 | 30 |
| Sorbosil®TC15 (ex PQ Corp.) | - | 1 |
| Sodium lauryl sulphate | 1.8 | 1.8 |

| | | |
|---|---|---|
| ⁽¹⁾ Precipitated calcium carbonate ex Maruo Calcium Company Limited, consisting of aragonite rods/needles with length 10 to 30 microns and diameter 0.5 to 1.0 micron. | | |

### Microscopy studies

The Example 1 paste was mixed with water at varying ratios of water to paste. The resulting mixtures were each evaluated for their interaction with human dentine. Samples of human dentine were brushed with each respective test mixture, by using a standard manual toothbrush and hand brushing for 30 seconds. The surface of each brushed dentine sample was analysed using SEM.

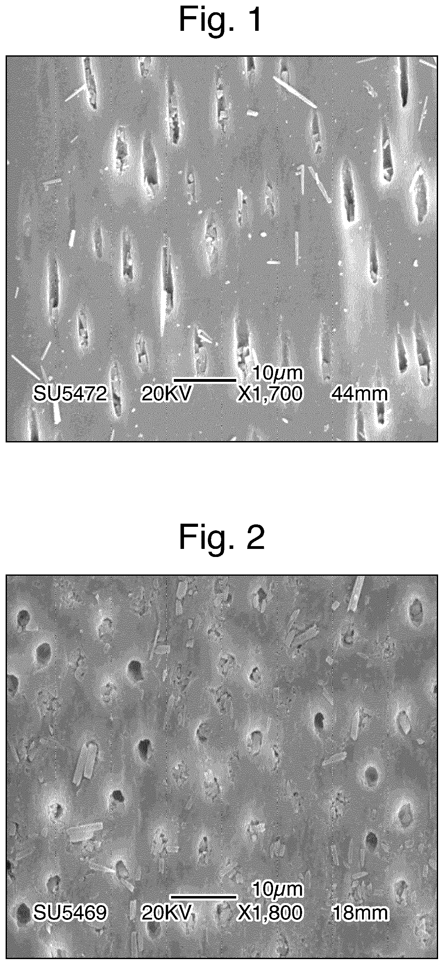

The appended Figure 1 shows an SEM image of the surface of a dentine sample brushed with a mixture of water and Example 1 paste in a 3:1 ratio of water:paste. The image shows the presence of particle fragments within tubules and some filling of tubules.

The appended Figure 2 shows an SEM image of the surface of a dentine sample brushed with a mixture of water and Example 1 paste in a 2:1 ratio of water:paste. This image shows a significant level of particle fragments found within tubules and tubule filling.

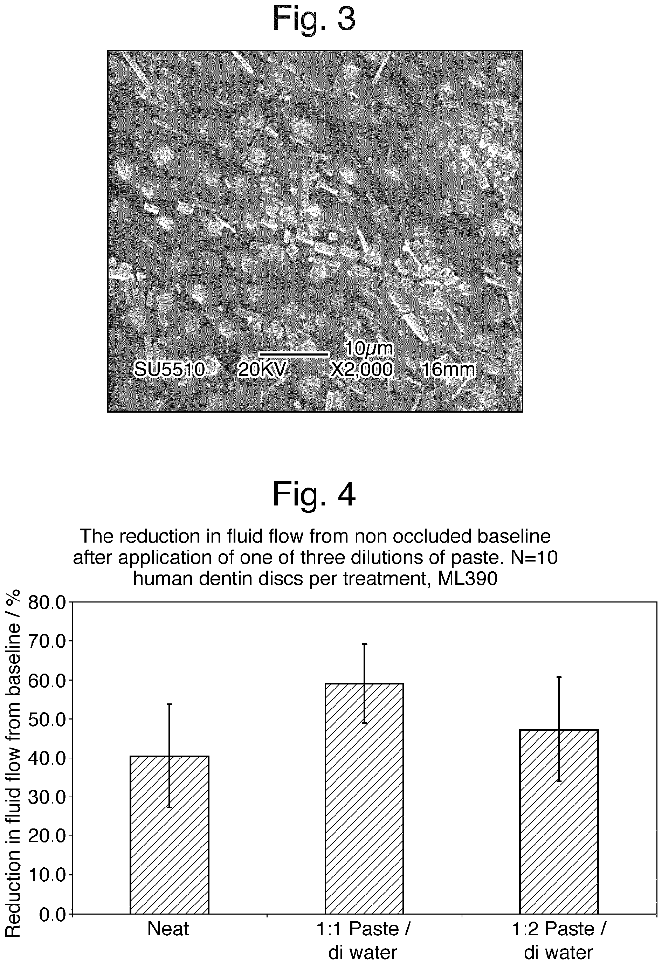

The appended Figure 3 shows an SEM image of the surface of a dentine sample brushed with a mixture of water and Example 1 paste in a 1:1 ratio of water:paste. This image shows tubules completely capped across almost the entirety of the sample. Also visible is an extensive, thin and smooth layer forming a barrier across the substrate surface. Even where this layer is intermittent or not present the tubules are still extensively filled and sealed.

The above results demonstrate how each test mixture is effective as a dentinal tubule occluding agent, with the 1:1 ratio of water:paste providing especially effective occlusion.

### Hydraulic conductance studies

The Example 1 paste was mixed with water at varying ratios of water to paste. The resulting mixtures were tested for their ability to reduce hydrodynamic fluid flow in the measurement of hydraulic conductance.

All test formulations were subjected to the standard hydraulic conductance procedure and the hydrodynamic flow measured.

### Protocol

Sound caries free human molars were sectioned with a wafering saw and dentin discs taken from between the crown and the pulp cavity. The discs were approximately 800 microns thick after sectioning. The discs were polished flat with 800 grit paper to a thickness of 500 microns and were finally polished with 2500 grit paper to give a flat polished dentin surface on both sides of the disc. The discs were then placed into 6% w/w citric acid and sonicated for five minutes.

The hydraulic conductance equipment was connected to a compressed air supply and the solvent chamber pressurised to 1.0 PSI. A dentin disc was placed into the holding chamber and EBSS Earles balanced salt solution (ex Sigma-Aldrich) passed through the system. An air bubble was introduced into the capillary tube via the input port and allowed to proceed along the capillary tube for a few seconds before being timed from a defined start point. A record was then made of the start position of the bubble and its progression timed over five minutes and the distance travelled measured at one minute intervals.

The test formulation was applied to the dentin disc using a Benda brush for 10 seconds and then left to soak in the formulation for two minutes. The disk was then rinsed with deionised water obtained by reverse osmosis. A second air bubble was introduced into the capillary tube and, after a brief pause, the distance travelled again measured over five minutes with the distance travel recorded at one minute intervals.

### Results

The flow rate was calculated from the displacement of liquid from the capillary tube over five minutes. The linearity of the flow rate was determined by calculating the r² of distance over time. Each occlusion value is the result of ten separate determinations. The average percentage occlusion for each of the test formulations is shown in Table 2 below, and the results shown graphically in the appended Figure 4.

**Table 2**

| **Test Product** | **% occlusion (SD)** |
|---|---|
| Example 1 paste (neat) | 40.6 (13.2) |
| A mixture of water and Example 1 paste in a 1:1 ratio of water:paste | 59.1 (10.2) |
| A mixture of water and Example 1 paste in a 1:2 ratio of water:paste | 47.4 (13.3) |

The above results demonstrate how each test mixture is effective as a dentinal tubule occluding agent, with the 1:1 ratio of water:paste providing especially effective occlusion.

## Claims

1. An anhydrous oral care dentifrice composition comprising 60 to 75 wt% glycerol (by weight based on the total weight of the dentifrice) and a particulate calcium carbonate composed of primary particles which are acicular and which have a length of 2 microns or greater, the composition being adapted to be applied to the surface of the teeth in a mixture with water; for the treatment of hypersensitivity arising in natural human teeth.

2. A composition according to claim 1, in which the particulate calcium carbonate is a crystalline calcium carbonate in which the crystals are acicular with a length of 10 microns or greater.

3. A composition according to claim 2, in which the crystals have a length ranging from 10 to 100 microns.

4. A composition according to claim 3, in which the crystals are acicular aragonite with a particle size of 10 to 100 microns by 0.5 to 4.0 microns.

5. A method of manufacture of a tooth treatment mixture for treating hypersensitivity arising in natural human teeth, the method comprising mixing an anhydrous oral care composition according to any one of claims 1 to 4 with water outside of the mouth.

6. A method according claim 5, in which the water is present as an aqueous activator capable of mixing with the anhydrous oral care composition and having an aqueous continuous phase comprising at least 50 wt% water (by weight based on the total weight of the aqueous activator).

7. A method according to claims 5 or 6 , in which the weight ratio of water to anhydrous oral care composition in the mixture ranges from 3:1 to 1:3.

8. A method according to claim 7, in which the weight ratio is 1:1.

## Patentansprüche

1. Wasserfreie Zahnputzmittelzusammensetzung für die Mundpflege, umfassend 60 bis 75 Gew.-% Glycerin (bezogen auf das Gesamtgewicht des Zahnputzmittels) und ein partikelförmiges Calciumcarbonat, gebildet mit primären Partikeln, die nadelförmig sind und die eine Länge von 2 Mikrometern oder mehr aufweisen, wobei die Zusammensetzung für das Auftragen auf die Oberfläche der Zähne in einer Mischung mit Wasser angepasst ist, um Überempfindlichkeit, die in natürlichen menschlichen Zähnen auftritt, zu behandeln.

2. Zusammensetzung nach Anspruch 1, in welcher das partikelförmige Calciumcarbonat ein kristallines Calciumcarbonat ist, in welchem die Kristalle nadelförmig mit einer Länge von 10 Mikrometern oder mehr sind.

3. Zusammensetzung nach Anspruch 2, in welcher die Kristalle eine Länge haben, die von 10 bis 100 Mikrometern reicht.

4. Zusammensetzung nach Anspruch 3, in welcher die Kristalle nadelförmiges Aragonit mit einer Partikelgröße von 10 bis 100 Mikrometern zu 0,5 bis 4,0 Mikrometern sind.

5. Verfahren zur Herstellung einer Zahnbehandlungsmischung zur Behandlung von Überempfindlichkeit, die in natürlichen menschlichen Zähnen auftritt, wobei das Verfahren das Mischen einer wasserfreien Mundpflegezusammensetzung nach irgendeinem der Ansprüche 1 bis 4 mit Wasser außerhalb des Mundes umfasst.

6. Verfahren nach Anspruch 5, in welchem das Wasser als wässriger Aktivator vorliegt, der zum Mischen mit der wasserfreien Mundpflegezusammensetzung fähig ist und eine wässrige kontinuierliche Phase aufweist, die mindestens 50 Gew.-% Wasser (bezogen auf das Gesamtgewicht des wässrigen Aktivators) umfasst.

7. Verfahren nach Anspruch 5 oder 6, in welchem das Gewichtsverhältnis von Wasser zu wasserfreier Mundpflegezusammensetzung in der Mischung von 3:1 bis 1:3 reicht.

8. Verfahren nach Anspruch 7, in welchem das Gewichtsverhältnis 1:1 beträgt.

## Revendications

1. Composition de dentifrice anhydre pour le soin oral comprenant de 60 à 75 % en masse de glycérol (en masse sur la base de la masse totale du dentifrice) et un carbonate de calcium particulaire composé de particules primaires qui sont aciculaires et qui présentent une longueur de 2 microns ou supérieure, la composition étant adaptée pour être appliquée à la surface des dents dans un mélange avec de l'eau ; pour le traitement d'hypersensibilité apparaissant dans des dents naturelles d'être humain.

2. Composition selon la revendication 1, dans laquelle le carbonate de calcium particulaire est un carbonate de calcium cristallin dans lequel les cristaux sont aciculaires avec une longueur de 10 microns ou supérieure.

3. Composition selon la revendication 2, dans laquelle les cristaux présentent une longueur de 10 à 100 microns.

4. Composition selon la revendication 3, dans laquelle les cristaux sont de l'aragonite aciculaire avec une taille de particule de 10 à 100 microns sur de 0,5 à 4,0 microns.

5. Procédé de fabrication d'un mélange pour le traitement de la dent destiné à traiter l'hypersensibilité apparaissant dans des dents naturelles d'être humain, le procédé comprenant le mélange d'une composition anhydre pour le soin oral selon l'une quelconque des revendications 1 à 4 avec de l'eau à l'extérieur de la bouche.

6. Procédé selon la revendication 5, dans lequel l'eau est présente comme un activateur aqueux capable de mélange avec la composition anhydre pour le soin oral et présentant une phase continue aqueuse comprenant au moins 50 % en masse d'eau (en masse rapportée à la masse totale de l'activateur aqueux).

7. Procédé selon la revendication 5 ou 6, dans lequel le rapport massique d'eau à composition anhydre pour le soin oral dans le mélange est de 3:1 à 1:3.

8. Procédé selon la revendication 7, dans lequel le rapport massique est de 1:1.
